# EUROPÄISCHE PATENTSCHRIFT

(11) **EP 1 267 306 B1**
(45) Veröffentlichungstag und Bekanntmachung des Hinweises auf die Patenterteilung: **20.07.2011**
(21) Anmeldenummer: 02010007.9
(22) Anmeldetag: 04.05.2002
(51) Int. Cl.: G06T 1/00, H04N 7/26

(54) **Verfahren zum Kennzeichnen von digitalen Videodaten**
Method for marking digital video data
Méthode pour repérer des données vidéo numériques

(30) Priorität: 31.05.2001 DE 10126435; 26.02.2002 DE 10208234
(43) Veröffentlichungstag der Anmeldung: 18.12.2002
(73) Patentinhaber: Deutsche Telekom AG, 53113 Bonn (DE)
(72) Erfinder: Schwenk, Jörg, Dr., 91239 Henfenfeld (DE)
(74) Vertreter: Puschmann, Heinz H.

(56) Entgegenhaltungen:
- JIANHAO MENG ET AL: "Embedding visible video watermarks in the compressed domain" IMAGE PROCESSING, 1998. ICIP 98. PROCEEDINGS. 1998 INTERNATIONAL CONFE RENCE ON CHICAGO, IL, USA 4-7 OCT. 1998, LOS ALAMITOS, CA, USA,IEEE COMPUT. SOC, US, Bd. 1, 4. Oktober 1998 (1998-10-04), Seiten 474-477, XP010308754 ISBN: 978-0-8186-8821-8
- GRIWODZ C ET AL: "PROTECTING VOD THE EASIER WAY" PROCEEDINGS OF THE ACM MULTIMEDIA 98. MM '98. BRISTOL, SEPT. 12 - 16, 1998; [ACM INTERNATIONAL MULTIMEDIA CONFERENCE], NEW YORK, NY : ACM, US, Bd. CONF. 6, 12. September 1998 (1998-09-12), Seiten 21-28, XP000977484 ISBN: 978-1-58113-036-2
- VAN SCHYNDEL R G ET AL: "Key independent watermark detection" MULTIMEDIA COMPUTING AND SYSTEMS, 1999. IEEE INTERNATIONAL CONFERENCE ON FLORENCE, ITALY 7-11 JUNE 1999, LOS ALAMITOS, CA, USA,IEEE COMPUT. SOC, US, Bd. 1, 7. Juni 1999 (1999-06-07), Seiten 580-585, XP010342789 ISBN: 978-0-7695-0253-3
- JIAN ZHAO: "Applying Digital Watermarking Techniques to Online Multimedia Commerce" PROCEEDINGS OF THE INTERNATIONAL CONFERENCE ON IMAGING SCIENCE,SYSTEMS AND APPLICATIONS, XX, XX, 30. Juni 1997 (1997-06-30), XP002221738

## Beschreibung

Die Erfindung betrifft ein Verfahren zum Kennzeichnen von digitalen Videodaten gemäß der im Anspruch 1 angegebenen Art sowie ein Verfahren zum Überwachen der Verteilung digitaler Videodaten über ein Datennetz gemäß Anspruch 16.

Zunehmend werden sowohl Fest- als auch Bewegtbilder (Videos) in digitaler Form erzeugt, übertragen sowie verarbeitet. Als Beispiele hierfür sind das digitale Fernsehen oder die Videoübertragung über das Internet zu nennen. Derartige Dienste werden sowohl im Privat-(Business-to-Consumer) als auch Geschäftsbereich (Business-to-Business) eingesetzt.

Als Geräte zum Darstellen der digitalen Bilder können digitale Fernsehgeräte, digitale Settop-Boxen, Personal-Computer, digitale Projektoren oder dergleichen Geräte verwendet werden.

Da gerade die Übertragung der digitalen Bild- und Videodaten große Bandbreiten erfordert, ist es besonders wichtig, die zu übertragenden digitalen Daten zu komprimieren. Zur Festbildkompression wird hierzu sehr häufig JPEG (Joint Pictures Expert Group) eingesetzt. Für die Kompression von Videodaten existieren unter anderem die MPEG-Standards (Motion Picture Experts Group), wie beispielsweise MPEG-1 und MPEG-2. Mit MPEG-1 läßt sich etwa Videorekorder-Qualität erreichen. MPEG-2 ermöglicht bereits HDTV (High Definition Television)-Qualität. Für die Übertragung von Videodaten über ISDN oder Modemstrecken wurde MPEG-4 entwickelt und standardisiert. MPEG-4 ermöglicht einen sehr hohen Kompressionsfaktor aufgrund der verhältnismäßig geringen Bandbreite von ISDN oder Modemstrecken.

Zudem sind im Computer-Bereich proprietäre Video-Codecs (Codierer-Decodierer) beispielsweise der Firmen RealNetworks (Produkt RealPlayer zum Abspielen von Videos), Microsoft (Produkt Microsoft Media Player zum Abspielen von Videos) und Apple (Produkt Qicktime zum Abspielen von Videos) verbreitet. Die Codecs ermöglichen verschiedene Kompressionsfaktoren, um an unterschiedliche Übertragungsbandbreiten anpassbar zu sein. Die von den Codecs erzeugten komprimierten Videodaten werden auch als Video-Streams bezeichnet, da sie häufig als "Datenstrom" zwischen verschiedenen Computern übertragen werden.

Aufgrund der Kompressionsverfahren ist es mittlerweile möglich, Daten mit audiovisuellem Inhalt über WANs (Wide Area Networks) wie beispielsweise das Internet zu verteilen. Dies ermöglicht jedoch auch, dass urheberrechtlich geschützte Werke sehr leicht unberechtigt verteilt werden können. Zur Verteilung von Audiodaten haben sich in letzter Zeit virtuelle "Tauschbörsen" im Internet etabliert, die aufgrund zahlreicher Urheberrechtsverletzungen mittlerweile in die Kritik gekommen sind. Für Videodaten existieren im Internet mittlerweile erste Tauschbörsen (c't 26/2001, S. 158 ff), erste derartige Börsen und es ist zu befürchten, dass aufgrund der mittlerweile sehr effizienten Kompressionsverfahren und der breitbandigen Internetzugänge, wie beispielsweise das von der Deutschen Telekom AG angebotene T-DSL (Telekom Digital Subscriber Line), in Zukunft Videodaten verstärkt unberechtigt auf eine derartige Weise verteilt werden. Bisher konnte von den Urhebern diese Art der Verteilung beispielsweise elektronisch nicht wirksam verhindert werden.

Um den Urheber eines Werkes bei digitalen Daten ermitteln zu können, wurden digitale Wasserzeichen entwickelt. Beispielsweise vertreibt die Firma Digimarc Software, mit der Urheber ihre (Fest-)Bilder mit digitalen Wasserzeichen versehen können. Zum Teil bleiben die digitalen Wasserzeichen auch nach einer Kompression, beispielsweise mittels JPEG, erhalten. Im Falle einer unberechtigten Kopie eines Bildes ist dann leicht feststellbar, von wem dieses Bild ursprünglich stammt. Digitale Wasserzeichen verhindern also grundsätzlich nicht den Zugriff, lassen jedoch Rückschlüsse auf den Urheber zu.

Sie weisen allerdings den gravierenden Nachteil auf, dass bei Bewegtbildern, also Videos, eine Rückverfolgung nach einer Kompression in der Regel nicht mehr möglich ist, da die statischen digitalen Wasserzeichen durch die hohen Kompressionsfaktoren von MPEG-Algorithmen im wesentlichen auf der Dynamik zwischen aufeinanderfolgenden Einzelbildern in einer Videosequenz basiert. MPEG liegt nämlich die Erkenntnis zugrunde, dass ein Bild für das menschliche Auge wichtige, normale und unwichtige Informationen enthält. Durch die Kompressionsverfahren wird nun ausgenutzt, dass lediglich wichtige und gegebenenfalls normale Informationen im komprimierten Datenstrom erhalten bleiben, unwichtige, meistens die statischen Informationen vom Kompressionsverfahren entfernt werden (sogenannte bewegungskompensierende Prädiktion) Dies hängt selbstverständlich von der Kompressionsrate ab: bei niedrigen Kompressionsraten werden nur die unwichtigen, bei hohen auch die normalen Informationen aus einem Bild entfernt.

Da jedoch Wasserzeichen die subjektive Qualität eines Bildes nicht beeinträchtigen dürfen, werden sie überwiegend als normale Information im Bild eingebettet. Da aber insbesondere bei der Übertragung über das Internet sehr hohe Kompressionsraten von Bild- oder Videodaten eingesetzt werden, ist eine Rückverfolgung zum Urheber der über das Internet übertragenen Videodaten in den meisten Fällen nicht mehr möglich.

Ein gattungsgemäßes, sämtliche Merkmale des Oberbegriffs des Anspruchs 1 aufweisendes Verfahren zum Kennzeichnen von digitalen Videodaten, die von einer Videoquelle digital erzeugt oder verarbeitet werden, ist aus der Veröffentlichung "Embedding visible video watermarks in the compressed domain "vgl. Jianhao Meng et al, IMAGE PROCESSING; 1998. ICIP. 98 PROCEEDINGS. 1998 INTERNATIONAL CONFE RENCE ON CHICAGO, IL, USA 4-7 OCT. 1998, los ALAMITOS, CA, USA, IEEE COMPUT. SOC, US, Bd. 1, 4. Oktober 1998 (1998-10-04), Seiten 474-477, XP010308754ISBN: 978-0-8186-8821-8.

Der vorliegenden Erfindung liegt die Aufgabe zugrunde, ein Verfahren zum Kennzeichnen von digitalen Videodaten sowie ein Verfahren zum Überwachen der Verteilung digitaler Videodaten vorzuschlagen, welche es auch bei einem hohen Kompressionsfaktor der digitalen Videodaten ermöglichen, eine in den Videodaten enthaltene Kennzeichnung zu ermitteln, welche Rückschlüsse auf die erzeugende und/oder verarbeitende Videoquelle ermöglicht, und für weitere Maßnahmen auswertbar ist.

Diese Aufgabe wird für das Verfahren zum Kennzeichnen von digitalen Videodaten durch die Merkmale des Anspruches 1 sowie für das Verfahren zum Überwachen der Verteilung digitaler Videodaten durch die Merkmale des Anspruches 16 gelöst.

Ein wesentlicher Gedanke der Erfindung ist es, einer die digitalen Videodaten digital erzeugenden und/oder verarbeitenden Videoquelle ein Identifikationsmerkmal zu zuordnen, welches beim Anzeigen des visuellen Inhalts der digitalen Videodaten als Grafik sichtbar wird. Hierdurch lässt sich die Vorrichtung, welche die digitalen Videodaten digital erzeugt und/oder verarbeitet hat, identifizieren. Der Vorteil liegt hier in dem erzielten Abschreckungseffekt gegenüber potentiell unberechtigten Verbreitern der digitalen Videodaten, der hohen Resistenz gegen Komprimierung, und der Echtzeitfähigkeit auf Client-Systemen. Unter Videoquelle wird hier sowohl eine analoge als auch digitale Videoquelle verstanden. Im Fall einer analogen Videoquelle ist vor einer Verarbeitung der digitalen Videodaten eine Digital/Analog-Wandlung erforderlich. Prinzipiell können mit der Erfindung auch analoge Videodaten eindeutig gekennzeichnet werden; in diesem Fall ist eine Analog/Digital-Wandlung erforderlich, um die Videodaten gemäß der Erfindung eindeutig zu kennzeichnen.

Das erfindungsgemäße Verfahren zum Kennzeichnen von digitalen Videodaten, die von einer Videoquelle digital erzeugt und/oder verarbeitet werden, ist demnach dadurch gekennzeichnet, dass der Videoquelle ein Identifikationsmerkmal zugeordnet ist. Mit diesem werden die digitalen Videodaten versehen. Beim Anzeigen des visuellen Inhalts der digitalen Videodaten wird es dann als Grafik sichtbar in einer von den digitalen Videodaten ausgefüllten Bildfläche eingeblendet und über diese Bildfläche bewegt. Im wesentlichen bietet diese Verfahren zwei Vorteile: einerseits kann ganz einfach durch Anzeigen des visuellen Inhalts der digitalen Videodaten und Auswerten der dann sichtbaren Grafik festgestellt werden, von welcher Videoquelle die Videodaten stammen. Zum Anderen verhindert die Bewegung der Grafik über die Bildfläche, das diese auch bei einem sehr hohen Kompressionsfaktor der digitalen Videodaten beispielsweise mit MPEG-4 nicht verschwindet.

Ein weiterer Vorteil des hier beschriebenen Verfahrens besteht darin, dass dieses Verfahren in Echtzeit auf Client-Systemen, wie z. B. Set-Top-Boxen oder PCs durchführbar ist. Dies ist für Videowasserzeichen aufgrund des hohen Rechenaufwands in der Regel noch nicht der Fall.

Vorzugsweise kann die Grafik gemäß einem vorgegebenen Bewegungsablauf über die Bildfläche bewegt werden. Dieser Bewegungsablauf ist dem Identifikationsmerkmal zugeordnet. In einer besonderen Ausführungsform ist die Grafik aus einem Punkt-Raster aufgebaut. Das Identifikationsmerkmal kann dabei sowohl dem Bewegungsablauf, als auch einer speziellen Anordnung der Punkte zugeordnet sein. Denkbar wäre beispielsweise, jedem Hersteller einer Videoquelle einen bestimmten Bewegungsablauf und/oder ein spezielles Punkt-Raster zuzuordnen. Damit ließe sich zumindest anhand der Auswertung des Bewegungsablaufes und/oder des Punkt-Rasters der Grafik der Hersteller der Videoquelle ermitteln.

Die digitalen Videodaten können entweder beim Erzeugen oder auch nach dem Erzeugen mit dem Identifikationsmerkmal versehen werden. Ersteres bietet sich beispielsweise bei einer Videokamera an, letzteres eher zur Nachbearbeitung von bereits vorhanden digitalen Videodaten, beispielsweise wenn ein Urheber dieser Videodaten sich zur Verbreitung über ein Datennetz entscheidet und diese davor kennzeichnen will.

Um den durch die digitalen Videodaten dargestellten Bildinhalt möglichst wenig zu stören, wird die Grafik vorzugsweise als Overlay über den visuellen Inhalt der digitalen Videodaten eingeblendet.

Da die Grafik von einem Betrachter des visuellen Inhalts der digitalen Videodaten kaum wahrnehmbar sein soll, kann sie aus dem visuellen Inhalt abgeleitet und/oder mit diesem verknüpft sein.

Beispielsweise ist für den neuen Harry Potter Film aus der umfassend angelegten Merchandising-Kampagne bekannt, dass eine Eule in dem Film zu sehen sein wird. Für den angesprochenen Harry Potter Film könnte daher die Grafik der Gestalt sein, dass eine entsprechende Eule als Grafik über die Bildfläche bewegt wird.

In diesem Fall wird die Grafik nur ein geschulter und aufmerksamer Betrachter erkennen. Eine Störung des Kunden durch das Einblenden einer solchen Grafik ist folglich weitestgehend auszuschließen.

Eine Feststellung, von welcher Videoquelle die digitalen Videodaten stammen, ist nun auf einfache Art und Weise möglich. Die Ableitung der Grafik aus dem visuellen Inhalt der digitalen Videodaten hat den weiteren Vorteil, dass sie vermeintlichen Hackern eher verborgen bleibt und somit ein Entfernen der Grafik erschwert wird.

In einer anderen Ausführungsform der Erfindung entspricht die Grafik einem Schriftzug. Dieser Schriftzug ist vorzugsweise einem Senderlogo nachempfunden. Da nach Beendigung einer Werbepause viele Sender heute für kurze Zeit ein Logo als eine Art "Markenname" einblenden, ist der Kunde an eine solche Grafik bereits gewöhnt. Es kann daher davon ausgegangen werden, dass das Einblenden einer einem Senderlogo nachempfundenen Grafik von den Betrachtern nicht als störend empfunden wird. Denkbar wäre auch, durch die Grafik Informationen zu vermitteln, beispielsweise durch die Einblendung des Filmtitels zu ausgewählten Zeitpunkten.

Die Videoquelle kann ein digitaler Videorecorder, eine digitale Videokamera, ein Personal-Computer mit einem Programm zum Erzeugen und/oder Verarbeiten digitaler Videodaten oder auch eine digitale Settop-Box, die digitale Videodaten erzeugen und/oder verarbeiten kann, sein. Vorgenannte Geräte sind nur eine beispielhafte Auflistung Videoquellen; selbstverständlich kann prinzipiell jedes Gerät als Videoquelle dienen, das digitale Videodaten erzeugen und/oder verarbeiten kann, beispielsweise auch ein analoger Videorekorder mit einem digitalen Eingang.

In einer bevorzugten Ausführungsform ist in einer digitalen Videokamera oder einem digitalen Videorecorder ein Sicherheitsmodul vorgesehen, dass eine Seriennummer erzeugt, die der Kamera beziehungsweise dem Recorder eindeutig zugeordnet ist. Diese Seriennummer dient dann als Individualitätsmerkmal für die digitale Videoquelle.

Bei einem Personal-Computer mit einem Netzwerk-Anschluss kann das Individualitätsmerkmal aus einer MAC-Adresse abgeleitet werden. Möglich ist auch die Ableitung des Individualitätsmerkmals aus einer Registry-Datenbank bei einem Personal-Computer, wenn dessen Betriebssystem eine derartige Datenbank aufweist. Hier ist anzumerken, dass sich grundsätzlich jede individuelle Ausstattung eines Personal-Computers für das Individualitätsmerkmal eignet. Nahezu jeder Personal-Computer besitzt eine unterschiedliche Software-Ausstattung. Aus dieser ließe sich ebenso ein Individualitätsmerkmal mit Hilfe eines dafür vorgesehenen Programms ableiten. Das Identitätsmerkmal kann auch mittelbar aus einem anderen Identitätsmerkmal, z. B. einem Zertifikat nach dem X.509-Standard (Beutelspacher, Schwenk, Wolfenstetter: Moderne Verfahren der Kryprographie, Vieweg Verlag; securemail.t-online.de), abgeleitet werden. Vertragsverhältnisse, die mit diesem anderen Identitätsmerkmal abgebildet werden, können so zur Kennzeichnung des Videos übernommen werden.

Besonders einfach gestaltet sich die Ableitung des Individualitätsmerkmals bei einer digitalen Set-Top-Box. Hierfür ist zum Betrieb fast immer eine Chipkarte vorgesehen, welche die Set-Top-Box personalisiert. Mit anderen Worten ist diese Chipkarte individuell, besitzt in der Regel individuelle Kennzeichnungen, die sich zum Ableiten des Individualitätsmerkmals ideal eignen. Das erfindungsgemäße Verfahren könnte in der Set-Top-Box beispielsweise wie folgt ablaufen:
Ein Content-Anbieter (z.B. die Firma Premiere World) sendet einen Premium-Film in Form digitaler Videodaten exklusiv an einen Besteller des Films. Dieser Film ist noch nicht als Leih- oder Kaufvideo erhältlich, sondern wurde bislang nur in Kinos gezeigt. Ein solcher Film ist für einen "Piraten", der illegal analoge oder digitale Kopien (z.B. auf VHS-Kassette, CD- oder DVD-ROM) verkaufen möchte, äußerst interessant. Die Gefahr einer unberechtigten Verbreitung des Films ist daher sehr groß. Der Film wird im Original verschlüsselt an die Set-Top-Box des Bestellers übertragen, zusammen mit einer Anweisung, einen individuellen Overlay auf dem Fernseher des Benutzers, der mit der Set-Top-Box verbunden ist, mit darzustellen. Diese Anweisung kann Zusatzinformationen zu dem zu erzeugenden Overlay enthalten wie z.B. die Bildschirmregion, in der sich die Overlay-Grafik bewegen soll, oder auch das Icon selbst, das bewegt werden soll (z.B. als JPEG-Bild). Das Icon kann in der Set-Top-Box auch so generiert werden, dass es selbst das Identitätsmerkmal enthält. Die Zusatzinformationen dienen dazu, den erzeugten Overlay an den Inhalt des Filmes anzupassen. Sie können während der Laufzeit des Films variiert werden. Die Set-Top-Box entschlüsselt den Originalfilm und liest die Anweisung, dass ein Overlay erzeugt werden muß. Die Set-Top-Box startet einen Prozess, der als Eingabe die Zusatzinfomationen und die individuelle Kennung der Set-Top-Box (z.B. die Chipkartennummer) erhält. Dieser Prozess generiert eine bewegte Grafik, mit der die Set-Top-Box die Filmdaten überlagert. Die Filmdaten plus die darüber gelegte bewegt Grafik werden von der Set-Top-Box ausgegeben. Somit ist der Film eindeutig mit den Individualitätsmerkmalen der Settop-Box des Bestellers versehen. Sollte der Besteller den Film auf einem Speichermedium ablegen, beispielsweise auf einer Videokassette, einer CD- oder DVD-ROM, ist später die Herkunft des Films anhand des Individualitätsmerkmals eindeutig nachvollziehbar. Tauchen nun illegale Kopien dieses Premium-Films auf, so können diese eingescannt und untersucht werden. Ein spezieller Bildanalyse-Algorithmus lokalisiert die Overlay-Grafik und bestimmt eine Folge von Positionen. Zusammen mit den bekannten Zusatzinformationen kann nun daraus die individuelle Kennung der Set-Top-Box ermittelt und weitere Schritte eingeleitet werden.

Vorzugsweise können die mit dem Identifikationsmerkmal versehenen digitalen Videodaten komprimiert werden. Wie bereits oben ausführlich erläutert, kann insbesondere bei einer in sich selbst animierten Grafik oder einer bewegten Grafik eine Kompression der Videodaten durchgeführt werden, ohne dass befürchtet werden muss, dass die Grafik durch die Kompression entfernt wird.

Ferner umfasst die Erfindung eine Vorrichtung zur Durchführung des erfindungsgemäßen Verfahrens, bei der eine Videoquelle Mittel zum Erzeugen eines Identifikationsmerkmals der Videoquelle aufweist.

Diese Mittel können ein Sicherheitsmodul umfassen, das ein Bauelement ist, welches einen Speicher zur Speicherung des Identifikationsmerkmals sowie einen Grafikprozessor zur Berechnung einer Grafik aus dem Identifikationsmerkmal und Einblendung der Grafik in der von den digitalen Videodaten ausgefüllten Bildfläche aufweist. Dieses Bauteil wird vorzugsweise eine integrierte Schaltung sein, die als den Speicher ROM (Read Only Memory) enthält.

Das Speichermodul kann in einen digitalen Videorecorder, eine digitale Videokamera, einer digitalen Settop-Box oder auch auf einer Grafik-Karte eines Personal-Computers eingebaut sein.

Die Erfindung weist in einer bevorzugten Ausführungsform einen weiteren wichtigen Aspekt auf, gemäß dem die unberechtigte Verbreitung digitaler Videodaten, welche mit dem erfindungsgemäßen Verfahren gekennzeichnet wurden, unterbunden werden kann. Die Unterbindung erfolgt hierbei in einem Datennetz, über das insbesondere digitale Videodaten übertragen werden.

Mindestens ein Server im Datennetz überwacht eingehende Datenströme auf digitale Videodaten, untersucht diese auf eine Kennzeichnung und steuert in Abhängigkeit vom Ergebnis der Untersuchung die Weiterleitung an einen Rechner eines Empfängers der digitalen Videodaten.

Konkret kann der Server nach dem Feststellen einer Kennzeichnung von digitalen Videodaten mittels einer Datenbank überprüfen, ob der Empfänger zum Empfangen der digitalen Videodaten berechtigt ist. Abhängig vom Ergebnis der Überprüfung kann der Server entweder die digitalen Videodaten weiterleiten oder aber auch die Weiterleitung abbrechen. Denkbar wäre beispielsweise, dass sich ein berechtigter Empfänger vorher in die Datenbank mittels eines Passwort-geschützten Zugangs eingetragen hat, beispielsweise nachdem er dem Urheber der digitalen Videodaten ein Entgeld bezahlt hat. Sobald die Videodaten an den Empfänger übertragen werden, schaltet sich der Server ein und überprüft eingehende digitale Videodaten auf die Kennzeichnung. Nach Feststellung einer Kennzeichnung sucht der Server in seiner Datenbank nach einem entsprechenden Eintrag für den Empfänger der Videodaten. Er wird den Empfänger finden, feststellen, dass dieser berechtigt ist zum Empfang und die Weiterleitung der digitalen Videodaten veranlassen.

Vorzugsweise wird der Server die Kennzeichnung von digitalen Videodaten mittels einer Mustererkennung durchführen. Da die Bildverarbeitung eine sehr speicher- und rechenintensive Verarbeitung erfordert, eignen sich zum Auffinden einer Kennzeichnung von digitalen Videodaten leistungsfähige Mustererkennungsverfahren.

In einer besonderen Ausgestaltung kann die Mustererkennung analysieren, ob und welchen Bewegungsablauf eine Grafik als Kennzeichnung der digitalen Videodaten besitzt. Daraus kann sie ermitteln, mit welcher digitalen Videoquelle die digitalen Videodaten erzeugt worden sind. Dies lässt im Falle einer unberechtigten Verbreitung der digitalen Videodaten Rückschlüsse auf die Videoquelle zu. Beispielsweise kann der Server, der die Mustererkennung durchführt, das Ergebnis der Analyse mit Zeit und Datum protokollieren für eine spätere Auswertung.

Weitere Vorteile, Merkmale und Anwendungsmöglichkeiten der vorliegenden Erfindung zum Kennzeichnen von digitalen Videodaten, die von einer Videoquelle erzeugt und/oder verarbeitet werden ergeben sich aus der nachfolgenden Beschreibung in Verbindung mit dem in der Zeichnung dargestelltem Ausführungsbeispiel.

Die Erfindung wird im folgenden, anhand der in der Zeichnung dargestellten Ausführungsbeispiele, näher beschrieben.

In der Beschreibung, in den Patentansprüchen, der Zusammenfassung und in der Zeichnung, werden die in der hinten angeführten Liste der Bezugszeichen verwendeten Begriffe und zugeordneten Bezugszeichen verwendet.

In der Zeichnung bedeutet:
- Fig. 1: ein Ausführungsbeispiel einer von digitalen Videodaten ausgefüllten Bildfläche und einer über die Bildfläche nach einem vorgegebenen Ablauf bewegten Grafik,
- Fig. 2: ein Ausführungsbeispiel eines Rechnernetzes mit einem Server, der eingehende Datenströme auf digitale Videodaten überwacht,
- Fig. 3: ein Ausführungsbeispiel einer digitalen Videoquelle mit einem Sicherheitsmodul zum Kennzeichnen digitaler Videodaten, und
- Fig. 4: ein Ausführungsbeispiel einer über die Bildfläche bewegten Grafik, die aus einem Punkt-Raster aufgebaut ist.

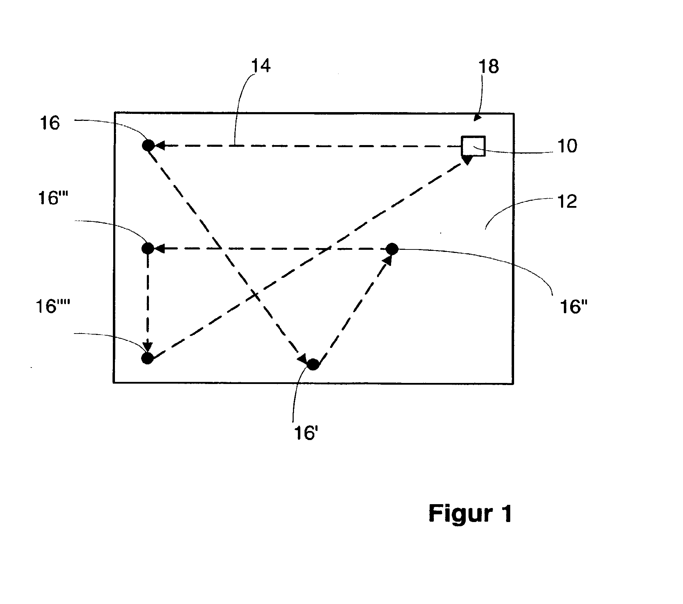

In Fig.1 ist eine Grafik 10 in einer von digitalen Videodaten ausgefüllten Bildfläche 12 dargestellt. Die Grafik 10 befindet sich an einer Ausgangsposition 18 im rechten oberen Eck der rechteckförmigen Bildfläche 12. Ausgehend von dieser Ausgangsposition 18 wird die Grafik 10 nach einem vorgegebenen Bewegungsablauf 14 über die Bildfläche 12 bewegt.

Der Bewegungsablauf 14 ist durch gestrichelte Pfeile dargestellt. Danach wird die Grafik 10 zu einem Eckpunkt 16 des Bewegungsablaufs 14 etwa in die linke obere Ecke der Bildfläche 12 bewegt. Von dem Eckpunkt 16 wird sie zu einem weiteren Eckpunkt 16' etwa in der Mitte am unterem Rand der Bildfläche 12 bewegt. Von dort wandert sie zu einem Eckpunkt 16'' nahe der Mitte der Bildfläche 12, von diesem auf einer horizontalen Linie zum linken Rand der Bildfläche 12 zu einem Eckpunkt 16''', um von dort zu einem Eckpunkt 16'''' in der linken unteren Ecke der Bildfläche 12 bewegt zu werden. Schließlich wandert die Grafik 10 von dem Eckpunkt 16'''' wieder zurück zur Ausgangsposition 18, von welcher der Bewegungsablauf erneut beginnt.

Der Bewegungsablauf 14 kann selbstverständlich auch anders gestaltet sein. Im Sinne der Erfindung ist er einer bestimmten digitalen Videoquelle zugeordnet. Für jeden Hersteller einer digitalen Videoquelle kann ein eindeutiger Bewegungsablauf 14 vorgegeben sein, sodass anhand einer Analyse des Bewegungsablaufs 14 der Grafik 10 über die Bildfläche 12 feststellbar ist, von welchem Hersteller die digitale Videoquelle zum Erzeugen der die Bildfläche 12 ausfüllenden digitalen Videodaten stammt.

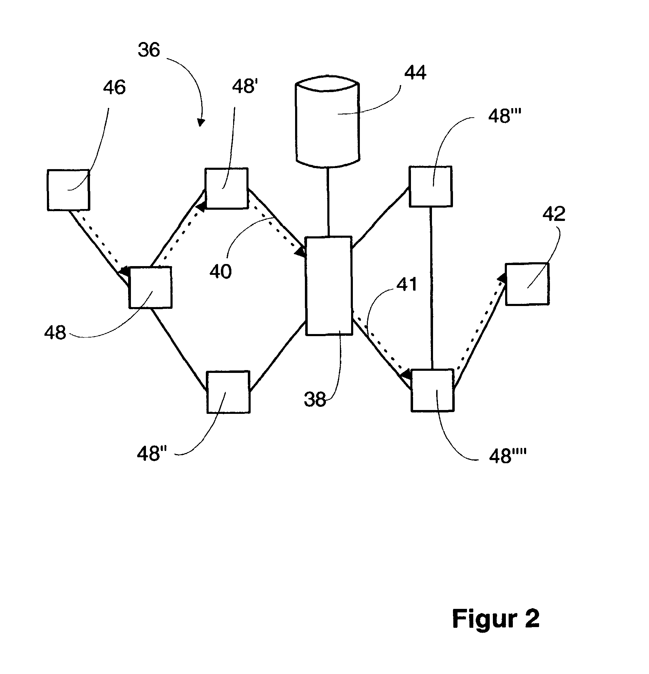

In Fig.2 ist ein Rechnernetz 36 dargestellt, in dem von einem Rechner 46 eines Senders digitale Videodaten an einen Rechner 42 eines Empfängers übertragen werden. Das Rechnernetz weist außer den beiden erwähnten Rechnern 42 und 46 weitere Rechner 48 - 48''' sowie einen Server 38 auf. Alle Datenströme vom Rechner 46 zum Rechner 42 laufen über den Server 38. Der Server 38 ist ferner mit einer Datenbank 44 verbunden.

Alle beim Server 38 eingehenden Datenströme 40 werden auf digitale Videodaten gescannt. Sobald der Server 38 im eingehenden Datenstrom 40 digitale Videodaten detektiert, untersucht er diese mittels eines Mustererkenners auf eine enthaltene Grafik, die ein Identifikationsmerkmal der digitalen Videoquelle, welche die digitalen Videodaten produziert hat, enthält.

Wird eine derartige Grafik erkannt, sucht der Server 38 in der Datenbank 44 nach einem Eintrag für den Rechner 42 des Empfängers der digitalen Videodaten. Wird kein Eintrag gefunden, blockiert der Server die Weiterleitung der digitalen Videodaten im eingehenden Datenstrom 40. Findet der Server 38 in der Datenbank 44 einen Eintrag für den Rechner 42 des Empfängers der digitalen Videodaten, zeichnet dies den Empfänger als zum Empfang berechtigt aus. Der Server 38 leitet dann die digitalen Videodaten an den Rechner 42 des Empfängers weiter; diese sind demnach im vom Server 38 ausgehenden Datenstrom 41 enthalten und können so den Rechner 42 des Empfängers erreichen.

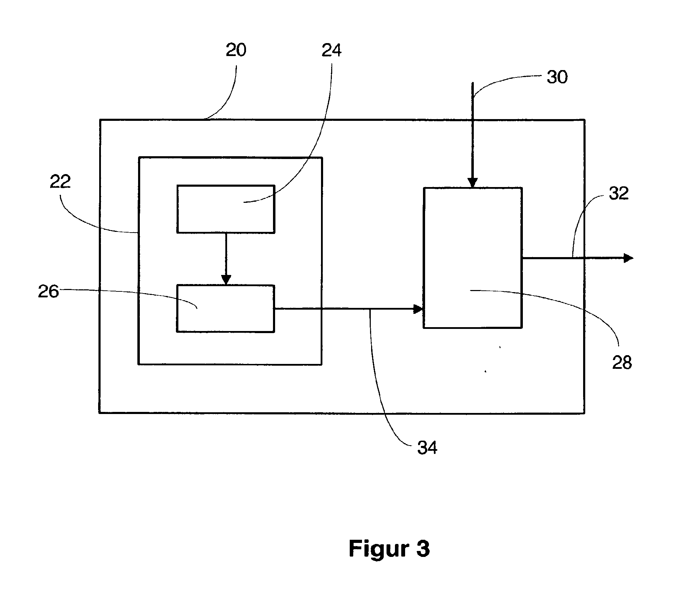

Fig.3 zeigt schließlich eine digitale Videoquelle 20, der Eingangsdaten 30 zur Bearbeitung zugeführt werden. Die Eingangsdaten 30 können beispielsweise elektrische Signale eines CCD (Charged Coupled Device)-Chips in einer digitalen Videokamera sein. In diesem Fall stellt die digitale Videoquelle 20 die Elektronik der digitalen Videokamera dar, welche die Eingangsdaten 30 in digitalen Videodaten 32 zum Aufzeichnen auf ein Magnetband oder dergleichen Speichermedium oder zum Ausgeben über eine digitale Schnittstelle umwandelt.

Ein in der digitalen Videoquelle 20 enthaltenes Sicherheitsmodul 22 weist einen Speicher 24 und einen Grafikprozessor 26 auf. In dem Speicher 24 ist eine die digitale Videoquelle 20 eindeutig identifizierende Seriennummer abgelegt, die beispielsweise von einer zentralen Vergabestelle an die Produzenten digitaler Videoquellen vergeben werden kann.

Der Grafikprozessor 26 liest aus dem Speicher 24 die Seriennummer aus, generiert daraus eine Grafik, die er mittels eines Grafik-Videosignals 34 einem Video-Prozessor 28 zuführt. Diesem werden ferner die Eingangsdaten 30 zugeführt. Aus den Eingangsdaten 30 und dem Grafik-Videosignal 34 generiert der Video-Prozessor 28 digitale Videodaten 32. Diese enthalten einerseits den in den Eingangsdaten enthaltenen audiovisuellen Inhalt als die eigentliche mit den digitalen Videodaten 32 zu übertragende oder zu speichernde Information und andererseits die von dem Grafik-Prozessor aus der der digitalen Videoquelle 20 zugeordneten Seriennummer erzeugten Grafik. Diese ist als kleine, sich über die digitale Bildfläche bewegende Overlay-Grafik sichtbar. Damit stellt die Overlay-Grafik ein die digitale Videoquelle 20 eindeutig identifizierendes Merkmal dar. Hierdurch kann die digitale Videoquelle 20 alleine anhand der digitalen Videodaten 32 identifiziert werden.

Die Erfindung zeichnet sich dadurch aus, dass digitale Videodaten eindeutig kennzeichenbar sind. Einer die digitalen Videodaten erzeugenden digitalen Videoquelle ist ein Identifikationsmerkmal, beispielsweise eine Seriennummer, zugeordnet, mit der digitale Videodaten versehen werden. Dies erfolgt durch eine Grafik, die beim Anzeigen des visuellen Inhalts der digitalen Videodaten in einem von diesem ausgefüllten Bildfläche sichtbar eingeblendet und über diese Bildfläche bewegt wird. Hierdurch wird eine Identifikation der digitalen Videoquelle ermöglicht. Durch die Bewegung der Grafik wird zudem verhindert, dass bei einer Kompression der digitalen Videodaten die Grafik entfernt wird.

Das erfindungsgemäße Verfahren zum Kennzeichnen von digitalen Videodaten wird im folgenden anhand einer Videobestellung über das Internet exemplarisch dargelegt: Eine solche Videobestellung über das Internet, Video-on-Demand, bietet beispielsweise die Firma "Arcor" seit geraumer Zeit unter der Webadresse "www.arcor.de" an.

Ein Besteller wählt sich über das Internet einen entsprechenden Film aus. Der Film wird nun komprimiert über das Internet an den Besteller gesendet. Mit dem Film wird auch eine Anweisung übertragen, dass bei Darstellung des bestellten Films auf der Bildfläche 12 eine Grafik 10 als Overlay eingeblendet werden soll. Zusätzliche Informationen, wie z. B. über die Art der Grafik 10, Angaben, ob die Grafik 10 ständig oder nur zu ausgewählten Zeitpunkten dargestellt werden soll, können mit der Anweisung übertragen werden. Wird der Film nun auf dem Personal-Computer des Bestellers abgespielt, erfolgt gleichzeitig die Auswertung der Anweisung. Die Anweisung startet einen Prozess, der als Eingabe die zusätzlichen Informationen und die individuelle Kennung des Personal-Computers erhält. Im vorliegenden Fall ist die individuelle Kennung dabei aus der MAC-Adresse des Personal-Computers abgeleitet. Auf der Bildfläche 12 erfolgt nun die Darstellung des bestellten Films und der darüber als Overlay gelegten Grafik 10. Der Film ist nun eindeutig mit den Individualisierungsmerkmalen des Personal-Computers der Bestellers versehen. Sollte der Besteller den Film auf einem Speichermedium ablegen, beispielsweise auf einer Videokassette, einer CD- oder DVD-ROM, ist später die Herkunft des Films anhand des Individualitätsmerkmal eindeutig nachvollziehbar.

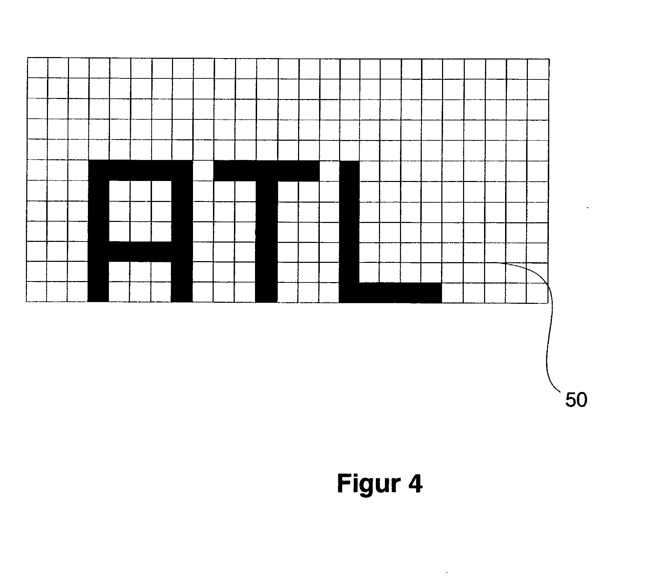

Als Grafik 10 wird im vorliegenden Fall, wie in Fig. 4 gezeigt, ein einem Senderlogo nachempfundener Schriftzug zu ausgewählten Zeitpunkten gemäß dem vorgegebenen Bewegungsablauf 14, 16, 16', 16", 16''', 16'''', 18 auf der Bildfläche 12 eingeblendet. Wie in Fig. 4 weiter dargestellt, ist die Grafik 10 dabei aus einem Punkt-Raster aufgebaut.

Tauchen nun zu einem späteren Zeitpunkten illegale Kopien von diesem Film auf, so kann aufgrund der Individualisierungsmerkmale die Herkunft des Films zurückverfolgt werden. Die Individualisierungsmerkmale können dabei sowohl in dem speziellen Bewegungsablauf 14, 16, 16', 16'', 16''', 16'''', 18 der Grafik 10 als auch in dem Punkt-Raster 50 enthalten sein.

### BEZUGSZEICHENLISTE

- 10: Grafik
- 12: Bildfläche
- 14: Bewegungsablauf
- 16: Eckpunkt
- 16': Eckpunkt
- 16": Eckpunkt
- 16"': Eckpunkt
- 18: Ausgangsposition
- 20: digitale Videoquelle
- 22: Sicherheitsmodul
- 24: Speicher
- 26: Grafikprozessor
- 28: Video-Prozessor
- 30: Eingangsdaten
- 32: digitale Videodaten
- 34: Grafik-Videosignal
- 36: Rechnernetz
- 38: Server
- 40: eingehender Datenstrom
- 41: ausgehender Datenstrom
- 42: Rechner eines Empfängers digitaler Videodaten
- 44: Datenbank
- 46: Rechner eines Senders digitaler Videodaten
- 48: Rechner
- 48': Rechner
- 48": Rechner
- 48"': Rechner
- 48"": Rechner
- 50: Punkt-Raster

## Patentansprüche

1. Verfahren zum Kennzeichnen von digitalen Videodaten, die von einer Videoquelle digital erzeugt oder verarbeitet werden und ein Identifikationsmerkmal beim Anzeigen des visuellen Inhalts der digitalen Videodaten als Grafik (10) sichtbar in einer von den digitalen Videodaten ausgefüllten Bildfläche (12) eingeblendet und über diese Bildfläche bewegt wird, **dadurch gekennzeichnet, dass** das Informationsmerkmal der Videoquelle zugeordnet ist und dass die Grafik (10) gemäß einem aus dem Identifikationsmerkmal abgeleiteten Bewegungsablauf (14, 16, 16', 16" , 16"', 16"", 18) über die Bildfläche (12) bewegt wird.

2. Verfahren nach Anspruch 1, **dadurch gekennzeichnet, dass** die Grafik (10) aus einem Punkt-Raster (50), welches aus dem Identifikationsmerkmal abgeleitet ist, aufgebaut ist.

3. Verfahren nach einem der vorangehenden Ansprüche, **dadurch gekennzeichnet, dass** die digitalen Videodaten beim oder nach dem Erzeugen mit dem Identifikationsmerkmal versehen werden.

4. Verfahren nach einem der vorangehenden Ansprüche, **dadurch gekennzeichnet, dass** die Grafik (10) als Overlay über den visuellen Inhalt der digitalen Videodaten eingeblendet wird.

5. Verfahren nach einem der vorangehenden Ansprüche, **dadurch gekennzeichnet, dass** die Grafik (10) ähnlich einem Onscreen-Display einen kleinen Teil der Bildfläche bedeckt.

6. Verfahren nach einem der vorangehenden Ansprüche, **dadurch gekennzeichnet, dass** die Grafik (10) aus dem visuellen Inhalt der digitalen Videodaten abgeleitet und/oder mit diesem verknüpft ist.

7. Verfahren nach einem der vorangehenden Ansprüche, **dadurch gekennzeichnet, dass** die Grafik (10) einem Schriftzug entspricht.

8. Verfahren nach einem der vorangehenden Ansprüche, **dadurch gekennzeichnet, dass** die Grafik (10) in sich selbst animiert ist.

9. Verfahren nach einem der vorangehenden Ansprüche, **dadurch gekennzeichnet, dass** die Videoquelle ein digitaler Videorekorder, eine digitale Videokamera, ein Personal-Computer mit einem Programm zum Erzeugen und/oder Verarbeiten digitaler Videodaten oder eine digitale Settop-Box, die digitale Videodaten erzeugt und/oder verarbeitet, ist.

10. Verfahren nach Anspruch 9, **dadurch gekennzeichnet, dass** in einer digitalen Videokamera oder einem digitalen Videorekorder ein Sicherheitsmodul vorgesehen ist, das als Identifikationsmerkmal für die digitale Videoquelle eine dieser eindeutig zugeordnete Seriennummer erzeugt.

11. Verfahren nach Anspruch 9, **dadurch gekennzeichnet, dass** bei einem Personal-Computer, der einen Netzwerk-Anschluss aufweist, das Identifikatiosmerkmal aus einer MAC-Adresse abgeleitet wird.

12. Verfahren nach Anspruch 9 oder 11, **dadurch gekennzeichnet, dass** bei einem Personal-Computer, dessen Betriebssystem eine Registry-Datenbank aufweist, das Identifikationsmerkmal aus der Registry-Datenbank abgeleitet wird.

13. Verfahren nach Anspruch 9 oder 11, **dadurch gekennzeichnet, dass** bei einem Personal-Computer das Identifikationsmerkmal mittelbar aus einem anderen Identifikationsmerkmal, insbesondere einem X.509-Zertifikat, abgeleitet wird.

14. Verfahren nach Anspruch 9, **dadurch gekennzeichnet, dass** bei einer digitalen SetTop-Box, für die eine Chipkarte zum Personalisieren vorgesehen ist, das Identifikationsmerkmal aus der Chipkarte abgeleitet wird.

15. Verfahren nach einem der vorangehenden Ansprüche, **dadurch gekennzeichnet, dass** die mit dem Identifikationsmerkmal versehenen digitalen Videodaten komprimiert werden.

16. Verfahren zum Überwachen der Verteilung digitaler Videodaten über ein Datennetz (36), die durch ein Verfahren nach einem der Ansprüche 1-15 **gekennzeichnet sind, dadurch gekennzeichnet, dass** mindestens ein Server (38) im Datennetz (36) eingehende Datenströme (40) auf digitale Videodaten überwacht, diese auf eine Kennzeichnung untersucht und davon abhängig die Weiterleitung an einen Rechner (42) eines Empfängers der digitalen Videodaten steuert, wobei eine Kennzeichnung von digitalen Videodaten mittels einer Mustererkennung erkannt wird, und die Mustererkennung die Grafik (10) analysiert und feststellt, ob und welchen Bewegungsablauf (14, 16, 16', 16", 16''', 16"", 18) die Grafik (10) der digitalen Videodaten besitzt und daraus ermittelt, mit welcher Videoquelle (20) die digitalen Videodaten erzeugt und/oder verarbeitet worden sind.

17. Verfahren nach Anspruch 16, **dadurch gekennzeichnet, dass** der Server (38) nach dem Feststellen einer Kennzeichnung von digitalen Videodaten mittels einer Datenbank (44) überprüft, ob der Empfänger zum Empfangen der digitalen Videodaten berechtigt ist, und abhängig vom Ergebnis der Überprüfung entweder die digitalen Videodaten weiterleitet oder die Weiterleitung abbricht.

## Claims

1. A method for labelling digital video data that is digitally generated or processed by a video source and in which, when the visual content of said digital video data is being displayed, an identification feature will become visible in the form of graphics (10) in a screen area (12) filled with said digital video data and will be moved across said screen area **characterized in that** the information feature is allocated to said video source and that the graphics (10) will be moved across said screen area (12) according to a motion sequence (14, 16, 16',16", 16"', 16"", 18) derived from said identification feature.

2. The method of claim 1 **characterized in that** the graphics (10) is composed of a dot matrix (50) which is derived from said identification feature.

3. The method of one of the preceding claims **characterized in that** the digital video data will be provided with said identification feature during or after their generation.

4. The method of one of the preceding claims **characterized in that** the graphics (10) will appear as an overlay over the visual content of said digital video data.

5. The method of one of the preceding claims **characterized in that** the graphics (10) - in the manner of an on-screen display - will cover a small portion of the screen area.

6. The method of one of the preceding claims **characterized in that** the graphics (10) is derived from the visual content of the digital video data and/or is linked with it.

7. The method of one of the preceding claims **characterized in that** the graphics (10) represents a lettering.

8. The method of one of the preceding claims **characterized in that** the graphics (10) is in itself animated.

9. The method of one of the preceding claims **characterized in that** the video source is a digital video recorder, a digital video camera, a personal computer with a program for generating and/or processing digital video data or a digital set top box which generates and/or processes digital video data.

10. The method of claim 9 **characterized in that** a security module is provided in a digital video camera or a digital video recorder which module will generate a serial number that is uniquely allocated to the digital video source as the identification feature for the digital video source.

11. The method of claim 9 **characterized in that** in a personal computer which has a network connection, the identification feature will be derived from a MAC address.

12. The method of claims 9 or 11 **characterized in that** in a personal computer whose operating system has a registry database, the identification feature will be derived from said registry database.

13. The method of claims 9 or 11 **characterized in that** in a personal computer, the identification feature will be derived indirectly from another identification feature, in particular an X.509 certificate.

14. The method of claim 9 **characterized in that** in a digital set top box for which a chip card has been provided for personalization, the identification feature will be derived from said chip card.

15. The method of one of the preceding claims **characterized in that** the digital video data provided with an identification feature will be compressed.

16. A method for monitoring the distribution of digital video data via a data network (36), said data being labelled by means of a method of one of claims 1-15 **characterized in that** at least one server (38) monitors incoming data streams (40) in the data network (36) for digital video data, checks whether such data has been labelled and depending on the result, controls the forwarding of said data to a computer (42) of a receiver of said digital video data, wherein a labelling of digital video data is recognized by means of pattern recognition, and said pattern recognition will analyse the graphics (10) and determine whether the graphics (10) of said digital video data includes a motion sequence (14, 16,16', 16",16"',16"", 18) and if so, what type of motion sequence, and will then determine based on this result which video source (20) was used for generating and/or processing said digital video data.

17. The method of claim 16 **characterized in that** once the digital video data has been found to be labelled, the server (38) will verify by means of a database (44) whether the receiver is authorized to receive said digital video data and - depending on the verification result - will then either forward said digital video data or interrupt its forwarding.

## Revendications

1. Méthode pour repérer des données vidéo numériques, qui sont créées ou traitées par une source vidéo numérique et une caractéristique d'identification est affichée de manière visible lors de l'affichage du contenu visuel des données vidéo numériques en tant que graphique (10) dans un écran (12) rempli par les données vidéo numériques et est déplacée sur cet écran, **caractérisée en ce que** la caractéristique d'information est affectée à la source vidéo et que le graphique (10) est déplacé selon une séquence de mouvements découlant de la caractéristique d'identification (14,16, 16', 16", 16"', 16"", 18) sur l'écran.

2. Méthode selon revendication 1, **caractérisée en ce que** le graphique (10) est constitué à partir d'une trame à points (50) laquelle découle d'une caractéristique d'identification.

3. Méthode selon l'une des revendications précédentes, **caractérisée en ce que** les données vidéo numériques sont pourvues de la caractéristique d'identification lors de la création ou après.

4. Méthode selon l'une des revendications précédentes, **caractérisée en ce que** le graphique (10) est affiché à l'écran en tant qu'overlay sur le contenu visuel des données vidéo numériques.

5. Méthode selon l'une des revendications précédentes, **caractérisée en ce que** le graphique (10) couvre comme un On-Screen Display une petite partie de l'écran.

6. Méthode selon l'une des revendications précédentes, **caractérisée en ce que** le graphique (10) découle du contenu visuel des données vidéos numériques et/ou est connecté à celui-ci.

7. Méthode selon l'une des revendications précédentes, **caractérisée en ce que** le graphique (10) correspond à un graphisme.

8. Méthode selon l'une des revendications précédentes, **caractérisée en ce que** le graphique (10) est animé en lui-même.

9. Méthode selon l'une des revendications précédentes, **caractérisée en ce que** la source vidéo est un magnétoscope numérique, une vidéo caméra numérique, un ordinateur personnel avec un programme pour créer et/ou traiter des données vidéo numériques ou un box settop numérique qui crée et / ou traite des données vidéo numériques.

10. Méthode selon revendication 9, **caractérisée en ce que** dans une vidéo caméra numérique ou un magnétoscope numérique, un module de sécurité est prévu, qui, en tant que caractéristique d'identification pour la source vidéo numérique, produit un numéro de série clairement affectée à celle-ci.

11. Méthode selon revendication 9, **caractérisée en ce que** dans le cas de l'ordinateur personnel, qui présente un raccordement au réseau, la caractéristique d'identification découle d'une adresse MAC.

12. Méthode selon revendication 9 ou 11, **caractérisé en ce que** dans le cas d'un ordinateur personnel, dont le système d'exploitation présente une base de données Registry, la caractéristique d'identification découle de la base de données Registry.

13. Méthode selon revendication 9 ou 11, **caractérisée en ce que** dans le cas d'un ordinateur personnel, la caractéristique d'identification découle indirectement d'une autre caractéristique d'identification, notamment un certificat X.509.

14. Méthode selon revendication 9, **caractérisé en ce que** dans le cas d'un box settop numérique, pour laquelle une carte à puce est prévue pour personnalisation, la caractéristique d'identification découle de la carte à puce.

15. Méthode selon l'une des revendications précédentes, **caractérisée en ce que** les données vidéo numériques pourvues de la caractéristique d'identification sont comprimées.

16. Méthode pour la surveillance de la répartition de données vidéo numériques par un réseau informatique (36), qui sont **caractérisées par** une méthode selon l'une des revendications 1 à 15, **caractérisée en ce qu'**un serveur (38) minimum dans le réseau informatique (36) surveille des flux de données entrants (40) sur des données vidéo numériques, les analyse pour trouver une caractéristique et en fonction de cela, commande le transfert vers l'ordinateur (42) d'un récepteur des données vidéo numériques sachant qu'un repérage de données vidéo numériques est reconnu au moyen d'une reconnaissance de forme, et la reconnaissance de forme analyse le graphique et détecte si la séquence de mouvement, et quelle séquence de mouvement (14, 16, 16', 16", 16"', 16"", 18) possède les données vidéo numériques et détermine à partir de cela avec quelle source vidéo (20) les données vidéo numérique ont été crées et / ou traitées.

17. Méthode selon revendication 16, **caractérisée en ce que** le serveur (38), après la détection d'une caractéristique de données vidéo numériques au moyen d'une base de données (44), vérifie si le récepteur est autorisé à recevoir les données vidéo numériques, et en fonction du résultat de la vérification, soit transfert les données vidéo numériques, soit annule la transmission.
